# EUROPEAN PATENT APPLICATION

(11) **EP 3 310 077 A1**
(43) Date of publication of application: **18.04.2018**
(21) Application number: 17192361.8
(22) Date of filing: 21.09.2017
(51) Int. Cl.: H04R 25/00, H04R 1/10, A61N 1/36, H01L 21/56, H05K 3/28, H05K 5/06, G06F 1/16, G06F 1/18, H05K 7/20

(54) **A HEARING DEVICE AND A METHOD FOR PROTECTING COMPONENTS OF A HEARING DEVICE**

(30) Priority: 13.10.2016 EP 16193666
(71) Applicant: Oticon A/S, 2765 Smørum (DK)
(72) Inventor: PEDERSEN, Troels Holm, 2765 Smørum (DK)
(74) Representative: William Demant

(57) **Abstract**

A hearing device includes at least one electrical component arranged in a cavity, where the cavity is filled at least partly with a filling material. The hearing device further comprises a cavity enclosure structure enclosing the cavity, which cavity enclosure structure comprises an injection hole and an opening, where the filling material extends from the injection hole towards the opening through the cavity so that the filling material is detectable through the opening.

## Description

### FIELD

The present disclosure relates to protective measures for components of hearing devices. More particularly, the disclosure relates to hearing devices, such as hearing aids, and methods for producing hearing devices, such that components of the hearing device are protected.

### BACKGROUND

Hearing devices, such as hearing aids, are often exposed to harsh conditions. Mechanical stress and corrosive liquids may affect components of the hearing aid or electro static discharge events may occur. It may be desired to protect the electronics from corrosive liquids, to protect the electronics from electro static discharge, to seal some connections, making them air or sound tight, and/or to fix some of the components to each other making the instrument more robust to impact. Therefore, there is a need to provide a solution that addresses at least some of the above-mentioned problems. The present disclosure provides at least an alternative to the prior art.

### SUMMARY

According to an aspect, a hearing device comprises at least one electrical component arranged in a cavity. The cavity is filled at least partly with a filling material. The hearing device further comprises a cavity enclosure structure enclosing the cavity. The cavity enclosure structure comprises an injection hole at least partly filled with the filling material and an opening. Further, the injection hole and the opening are formed between an outside surface of the cavity enclosure structure and the cavity. The filling material extends at least from the injection hole towards the opening through the cavity so that the filling material is detectable through the opening.

By filling at least a part of the cavity with a filling material, a protection of at least the electrical component against corrosive fluids and/or electro static discharge may be improved. By using a cavity enclosure structure with an injection hole and an opening, the filling material may be brought into the cavity with low effort and high accuracy. In this way, the reliability of the hearing device may be improved and/or the costs may be reduced. A coating of components in the cavity may be enabled. In more detail, the injection hole may enable a process of introducing the filling material into the cavity. In addition, the opening in the housing structure provides at least a ventilation structure ventilating the cavity during the process of filing the housing with the filing material, and also may provide a suitable inspection hole through which the amount of filing the cavity can be followed.

According to another aspect, a method for producing a hearing device comprises providing an at least partly assembled hearing device comprising an electrical component arranged in a cavity and a cavity enclosure structure enclosing the cavity. The cavity enclosure structure comprises an injection hole and an opening. Further, the injection hole and the opening are formed between an outside surface of the cavity enclosure structure and the cavity. Further, the method comprises introducing filling material through the injection hole into the cavity at least until the filling material is detectable through the opening.

By injecting the filling material into the cavity through an injection hole until the filling material reaches the opening, it can be easily detected when at least a desired part of the cavity is filled. At the same time the first opening may provide an air ventilation of the cavity. In this way, a hearing device may be manufacturable with high reliability and/or high yield and/or at low costs and/or faster production time. By using an injection hole, the position of the injection hole at the hearing device is known so that an automatization of the injection of the filling material may be enabled.

For example, the effects and technical solutions related to the hearing aid device may also be understood to automatically be applicable for the described method.

### BRIEF DESCRIPTION OF DRAWINGS

The aspects of the disclosure may be best understood from the following detailed description taken in conjunction with the accompanying figures. The figures are schematic and simplified for clarity, and they just show details to improve the understanding of the claims, while other details are left out. Throughout, the same reference numerals are used for identical or corresponding parts. The individual features of each aspect may each be combined with any or all features of the other aspects. These and other aspects, features and/or technical effect will be apparent from and elucidated with reference to the illustrations described hereinafter in which:
Figure 1 illustrates a schematic cross section of a part of a hearing device according to an embodiment of the disclosure;
Figure 2A illustrates a schematic view of another hearing device according to an embodiment of the disclosure;
Figure 2B illustrates another schematic view of the hearing device of Fig. 2A according to an embodiment of the disclosure;
Figure 3 illustrates a schematic view of a cavity enclosure component of the hearing device of Fig. 2A.
Figure 4 illustrates another schematic view of the cavity enclosure component of the hearing device of Fig. 2A.
Figure 5 illustrates a schematic view of a substrate and the cavity enclosure component of the hearing device of Fig. 2A.
Figure 6 illustrates a schematic view of a substrate and a cavity enclosure component of another hearing device.
Figure 7 illustrates another schematic view of the substrate and the cavity enclosure component of Fig. 6.
Figure 8 illustrates another schematic view of the substrate and the cavity enclosure component of Fig. 6.
Figure 9 illustrates a schematic cross section of a cavity of a hearing device;
Figure 10 illustrates a schematic cross section of an inspection hole of a hearing device; and
Figure 11 illustrates a flow chart of a method for forming a hearing device according to an embodiment of the disclosure.

### DETAILED DESCRIPTION

The detailed description set forth below in connection with the appended drawings is intended as a description of various configurations. The detailed description includes specific details for the purpose of providing a thorough understanding of various concepts. However, it will be apparent to those skilled in the art that these concepts may be practiced without these specific details. Several aspects of the apparatus and methods are described by various blocks, functional units, modules, components, circuits, steps, processes, algorithms, etc. (collectively referred to as "elements"). Depending upon particular application, design constraints or other reasons, these elements may be implemented using electronic hardware, computer program, or any combination thereof.

The electronic hardware, such as electronic components of a hearing aid according to the disclosure, may include microprocessors, microcontrollers, digital signal processors (DSPs), field programmable gate arrays (FPGAs), programmable logic devices (PLDs), gated logic, discrete hardware circuits, and other suitable hardware configured to perform the various functionality described throughout this disclosure. Computer program shall be construed broadly to mean instructions, instruction sets, code, code segments, program code, programs, subprograms, software modules, applications, software applications, software packages, routines, subroutines, objects, executables, threads of execution, procedures, functions, etc., whether referred to as software, firmware, middleware, microcode, hardware description language, or otherwise.

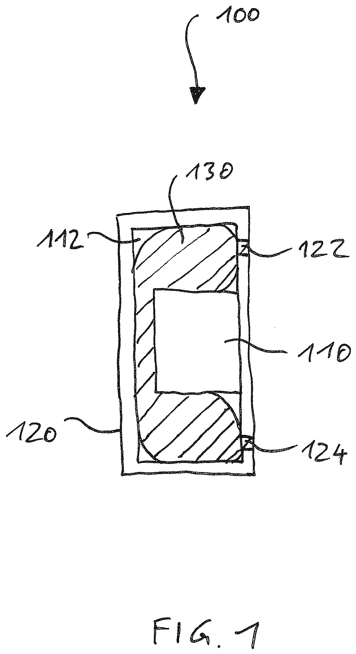

Fig. 1 shows a schematic cross section of a part of a hearing device, such as a hearing aid 100, according to an embodiment. The hearing aid 100 comprises at least one electrical component 110 arranged in a cavity 112. The cavity 112 is filled at least partly with a filling material 130. The hearing aid further comprises a cavity enclosure structure 120 enclosing the cavity 112. The cavity enclosure structure 120 comprises an injection hole 122 at least partly filled with the filling material 130 and an opening 124. Further, the injection hole 122 and the opening 124 are formed between an outside surface of the cavity enclosure structure 120 and the cavity. The filling material 130 extends at least from the injection hole 122 towards the opening 124 through the cavity 112 so that the filling material is detectable through the opening 124.

For example, during manufacturing of the hearing aid 100, the filling material 130 can be injected into the cavity 112 through the injection hole 122 until the filling material 130 reaches the (first) opening 124 (e.g. an inspection hole or a ventilation gap) or the proximity of the opening 124 so that the filling material is detectable through the opening 124 (e.g. by a detector of the injection system or by visual inspection). For example, the filling material 130 extends from the injection hole 122 to the opening 124 through the cavity and fills at least partly the injection hole 122 and optionally also the opening 124. Depending on the geometry of the cavity and/or the positions of the injection hole 122 and the opening 124, only a minor part of the cavity, a large portion of the cavity or substantially the complete cavity may be filled with the filling material 130.

For example, the protection of the electrical component 110 may be improved, if most of the space or substantially the complete space of the cavity unoccupied by components (e.g. plurality of electrical components, printed circuits boards and/or wiring structures) of the hearing aid is filled with filling material 110. For example, the filling material fills more than 70% (or more than 80% or more than 90%) of a space of the cavity unoccupied by components of the hearing aid.

For example, the injection hole 122 and the (first) opening 124 may be located far away from each other to enable a substantially complete filling of the cavity. For example, a distance between the injection hole 122 and a point within the cavity furthest away from the injection hole 122 is smaller than twice (or smaller than 1.5 times or smaller than 1.2 times) a distance between the injection hole 122 and the opening 124. For example, instead of or additional to the distance between the injection hole 122 and the inspection hole 124, a substantially complete filling may arise, if the geometry of the cavity 112 is such that the filling material 130 may flow along the inner structure of the cavity and potentially reach the opening 124 (e.g. the inspection hole), where one will stop with injecting the filling material. For example, the injection hole 122 may be located in a first end of the cavity and the opening 124 in a second end of the cavity opposite to the second end. This may enable that the filling of the cavity reaches into the entire cavity, due to the filling process being stopped when the filling material can be visual identified in the second end of the cavity through the opening.

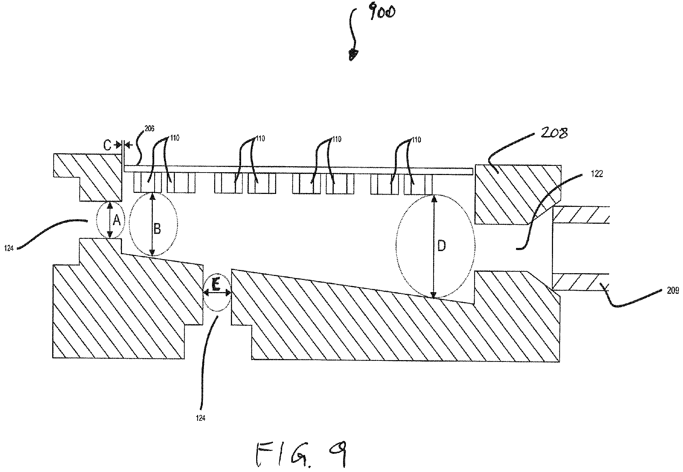

For example, the injection hole 122 may be a substantially circular or rectangular opening through the cavity enclosure structure 130. A geometry and dimension of the injection hole 122 may be selected based on a geometry and dimension of an injection tool (e.g. needle of a syringe or an automatic injection system) used to inject the filling material 130. For example, the injection hole 122 may comprise a cross section with a maximal extension (e.g. largest distance between opposite sides of the cross section, for example, diameter of a circle or length of a rectangle) smaller than 2mm (or smaller than 1mm or smaller than 0.7mm). For example, the injection hole 122 may be formed with a substantial conical geometry, such that the largest diameter of the injection hole is formed at the outer surface of the cavity enclosure structure 120 and the injection hole narrows in diameter towards the interior of the cavity 112. Further details on possible geometries of the injection hole 122 are shown in Fig. 9, for example. This narrowing injection hole aids in leading the filling material into the cavity due to the effect of capillary forces, which are prone to follow narrow paths.

For example, the opening 124 may be an inspection hole (e.g. substantially circular or rectangular hole) or an inspection gap (e.g. thin slot shape). The opening 124 may be a first type opening, which may be used for inspection of the amount of filling material 130 which has reached into the cavity. However, such first-type opening may also be used for ventilation alone and/or have a double function and used for both ventilation and visual inspection purposes, for example. The opening 124 may be at least partly filled with the filling material 130, if the filing material reaches the opening 124 during introduction of the filling material 130 through the injection hole, or may be free of the filling material, if the injection of the filling material 130 through the injection hole 122 is stopped before the filling material 130 reaches the opening 124, but is already detectable through the opening 124, for example.

A geometry and dimension of the opening 124 may be selected so that no liquid filling material leaks out of the opening 124 due to capillary forces acting during manufacturing. For example, the opening 124 may comprise a cross section with a minimal extension (e.g. smallest distance between opposite sides of the cross section, for example, diameter of a circle or width of a rectangle) smaller than 2mm (or smaller than 1mm or smaller than 0.7mm). For example, the opening 124 may be configured with a path extending from the interior of the cavity 112 to an outside of the cavity enclosure structure 120 so that the path narrows in cross-section area from the interior of the cavity to an end facing a transition to a last part of the opening 124 facing the outside of the cavity enclosure structure 120 and the transition is formed as a (e.g. step-shaped) widening of the cross-section of the path. For example, the (first) opening 124 (e.g. the inspection hole) narrows from diameter B to diameter A and then expands to the outside surface of the cavity enclosure structure 120 (e.g. part of the housing structure of the hearing aid). In this way, capillary forces aids in forcing the filling material to seek into the inspection hole, but only until the transition point and the larger (e.g. sharp) transition may create a capillary trap, which may cause a stop to the flow of the material. In case some material keeps flowing, the widening may provide a chamber, which contains the overflow of material, for example.

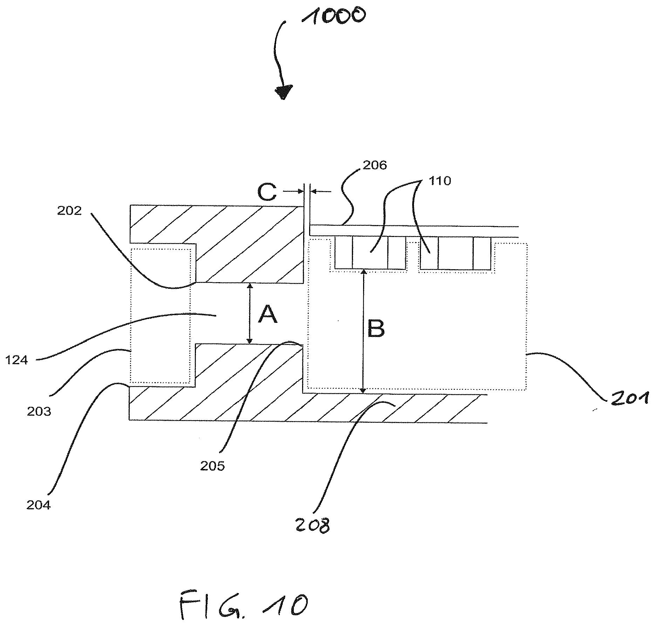

For example, the injection hole 122 and the opening 124 may comprise different geometries towards the outside surface. The injection hole 122 may comprise a conical shape while the opening 124 may comprise a step-shaped increase of the diameter of the opening 124 at the outside surface, for example. Further details on possible geometries of the opening 124 are shown in Fig. 9 and 10, for example.

Optionally, the opening 124 is one inspection opening of a plurality of the inspection openings of the cavity enclosure structure 120. The plurality of inspection openings may be distributed over the cavity so that the success of a substantially complete filling of the cavity can be checked at various positions of the cavity, for example. The filling material may extend through the cavity from the injection hole 122 to each inspection opening 124 of the plurality of the inspection openings.

For example, a path extends in the interior of the cavity 112 between the injection hole 122 and the opening and the path at a part of the cavity located close to the injection hole (e.g. closer than to the opening) 122 may comprise a first height and the path at a part of the cavity located close to the opening 124 (e.g. closer than to the injection hole) comprises a second height. For example, the second height is smaller than the first height (e.g. second height is less than 95%, less than 90% or less than 80% of the first height) and the cavity 112 narrows along the path from the first height to the second height. In this way, the filling material may be sucked towards the opening 124 during injection due to capillary forces, for example. By narrowing the path from the injection hole to the end of the cavity may provide a capillary effect, where the filling material by capillary forces will be forced towards the narrowing end of cavity opposite to the injection holes, for example. These capillary forces may work substantially alone to distribute the filling material in the cavity. These capillary forces may aid in preventing the filling material from spilling out of the holes due to pressure forces from the injection, as the case may be if only pressure forces would be used. The "path" could be one or more narrowing paths, defined by a set of components lying in close connection, but separated by small gaps, which gaps may form a small narrow path through which the capillary forces will tend to guide filling material through, for example.

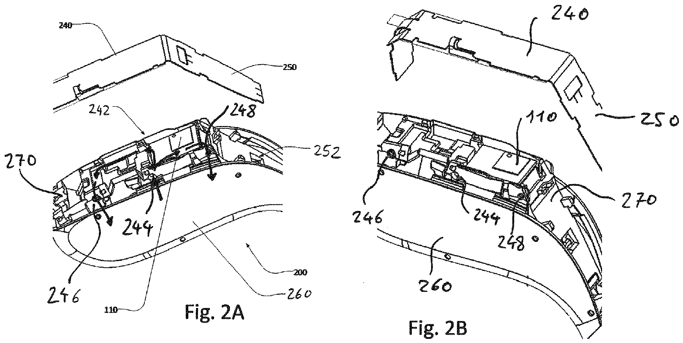

The position of the injection hole 122 and the positions of the plurality of inspection openings may be selected so that the length of the paths from the injection hole 122 to the different inspection holes may differ by less than 50% (or less than 30% or less than 10%) of an average length of the paths. In this way, the filling material may reach each inspection opening at a similar time. The paths form the injection hole 122 to the inspection openings may depend on the position and geometry of the electrical component 110 and the geometry of the cavity enclosure structure 120. The position of the injection hole 122 and/or the positions of the plurality of inspection openings and/or the geometry of the cavity enclosure structure 120 and the electrical component 110 may be selected so that the paths differ from each other along a significant part of the paths. For example, at least the cavity enclosure structure 120 and the electrical component 110 may comprise a geometry so that, during injection of the filling material 130, at least 50% (or at least 70%) of a preferred path of the filling material 130 form the injection hole 122 to a first inspection opening 124 of the plurality of the inspection openings differs from a preferred path of the filling material 130 from the injection hole 122 to a second inspection opening of the plurality of the inspection openings. A preferred path may be a path between the injection hole 122 and an inspection opening taken by a main portion of the filling material 130 on the way from the injection hole 122 to the opening. An example of a different preferred path in a cavity of a hearing aid is indicated by arrows in Fig. 2A.

Optionally, at least one of the injection hole 122 and the opening 124 is located in a recess (or depression or deepening) at the outside surface of the cavity enclosure structure 120. The recess may form a reservoir for collecting or absorbing droplets sitting at the end of the injection tool or leaking out of the inspection hole during injection of the filling material 130. For example, the recess may comprise a diameter of more than 1.2 times a diameter of the injection hole 122 or the opening 124 and less than 2 times the diameter of the injection hole 122 or the opening 124. The recess may comprise a depth of more than 20% of a depth of the injection hole 122 or the opening 124 and less than 50% of the depth of the injection hole 122 or the opening 124, for example.

The filling material 130 may comprise a liquid state during dispensing into the cavity through the injection hole 122 and may be a solid or elastic material after curing or hardening in the cavity. The filling material 130 may be a coating material. For example, the filling material may be chosen from an epoxy, silicone coating or e.g. a polyurethane material. For example, the filling material is a liquid state material which hardens by a 2-components reaction, reaction with moist in the air and/or by a dehydration process. A liquid state filling material, which may be prone to seek capillary traps may be suitable for the filling process.

The cavity 112 may be a completely enclosed space except for one or more injection holes and one or more openings, such as inspection and/or ventilation openings. For example, the cavity is enclosed so that, during manufacturing, the filling material can leak out of the cavity through the one or more injection holes and one or more inspection openings only.

For example, the electrical component 110 may be configured to enable or take part in the generation of an audio signal perceptible by a user of the hearing aid. For example, the electrical component 110 may be at least one of a signal processor circuit, a filter circuit, an analog-to-digital converter circuit, a memory circuit, a sensor circuit, a digital-to-analog converter circuit, a receiver circuit, a transmitter circuit, a transceiver circuit, a capacitor and an inductor.

For example, the at least one electrical component 110 may be arranged on a substrate (e.g. PCB) and the substrate may form a second cavity enclosure component of the cavity enclosure structure 120 fixed to a first cavity enclosure component of the cavity enclosure structure 120.

The cavity enclosure components of the cavity enclosure structure 120 together may enclose the cavity 112. The injection hole 122 and/or the opening 124 may be arranged in a cavity enclosure component being part of the housing structure of the hearing aid and/or the substrate carrying the at least one electrical component 110 or another substrate (e.g. PCB) carrying one or more further electrical components.

For example, the cavity enclosure structure 120 may comprise a plurality of cavity enclosure components (e.g. one-piece parts of a multi-piece cavity enclosure structure) assembled to result in the cavity enclosure structure 120. For example, one or more cavity enclosure components of the cavity enclosure structure 120 may be a part of or may form a housing structure (e.g. chassis, case, enclosure, cabinet or shell structure) of the hearing aid 100. For example, the cavity enclosure components of the cavity enclosure structure 120 may be plastic components and/or metal components and/or compound components. For example, the injection hole 122 and the opening 124 may be formed at the same cavity enclosure component (e.g. one-piece component, for example, a plastic component) of the cavity enclosure structure 120. Alternatively, the opening 124 may be a gap (e.g. ventilation gap) between two cavity enclosure components of the cavity enclosure structure 120.

The outside surface of the cavity enclosure structure 120 may be an externally accessible surface of the hearing aid 100 or an internal surface of the hearing aid 100 enclosed or covered after injection of the filling material 130.

Optionally, a further (second) electrical component may be arranged in a further (second) cavity filled at least partly with a filling material. A further (second) cavity enclosure structure 120 of the hearing aid 100 may enclose the further (second) cavity and the further cavity enclosure structure 120 may comprise a further (second) injection hole at least partly filled with the filling material and a further (second) opening. The filling material may extend at least from the further injection hole towards the further opening through the further cavity so that the further filling material is detectable through the further opening.

A hearing device may include a hearing aid that is adapted to improve or augment the hearing capability of a user by receiving an acoustic signal from a user's surroundings, generating a corresponding audio signal, possibly modifying the audio signal and providing the possibly modified audio signal as an audible signal to at least one of the user's ears. The "hearing device" may further refer to a device such as an earphone or a headset adapted to receive an audio signal electronically, possibly modifying the audio signal and providing the possibly modified audio signals as an audible signal to at least one of the user's ears. Such audible signals may be provided in the form of an acoustic signal radiated into the user's outer ear, or an acoustic signal transferred as mechanical vibrations to the user's inner ears through bone structure of the user's head and/or through parts of middle ear of the user or electric signals transferred directly or indirectly to cochlear nerve and/or to auditory cortex of the user.

The hearing device is adapted to be worn in any known way. This may include i) arranging a unit of the hearing device behind the ear with a tube leading air-borne acoustic signals into the ear canal or with a receiver/ loudspeaker arranged close to or in the ear canal such as in a Behind-the-Ear type hearing aid, and/ or ii) arranging the hearing device entirely or partly in the pinna and/ or in the ear canal of the user such as in a In-the-Ear type hearing aid or In-the-Canal/ Completely-in-Canal type hearing aid, or iii) arranging a unit of the hearing device attached to a fixture implanted into the skull bone such as in Bone Anchored Hearing Aid or Cochlear Implant, or iv) arranging a unit of the hearing device as an entirely or partly implanted unit such as in Bone Anchored Hearing Aid or Cochlear Implant. In addition to the above mentioned devices, the general concept of the method described herein could similarly be implemented in other applications, such as hearing instrument accessory or other similar devices that needs protection from its environment.

A "hearing system" refers to a system comprising one or two hearing devices, and a "binaural hearing system" refers to a system comprising two hearing devices where the devices are adapted to cooperatively provide audible signals to both of the user's ears. The hearing system or binaural hearing system may further include auxiliary device(s) that communicates with at least one hearing device, the auxiliary device affecting the operation of the hearing devices and/or benefitting from the functioning of the hearing devices. A wired or wireless communication link between the at least one hearing device and the auxiliary device is established that allows for exchanging information (e.g. control and status signals, possibly audio signals) between the at least one hearing device and the auxiliary device. Such auxiliary devices may include at least one of remote controls, remote microphones, audio gateway devices, mobile phones, public-address systems, car audio systems or music players or a combination thereof. The audio gateway is adapted to receive a multitude of audio signals such as from an entertainment device like a TV or a music player, a telephone apparatus like a mobile telephone or a computer, a PC. The audio gateway is further adapted to select and/or combine an appropriate one of the received audio signals (or combination of signals) for transmission to the at least one hearing device. The remote control is adapted to control functionality and operation of the at least one hearing devices. The function of the remote control may be implemented in a SmartPhone or other electronic device, the SmartPhone/ electronic device possibly running an application that controls functionality of the at least one hearing device.

In general, a hearing device includes i) an input unit such as a microphone for receiving an acoustic signal from a user's surroundings and providing a corresponding input audio signal, and/or ii) a receiving unit for electronically receiving an input audio signal. The hearing device further includes a signal processing unit for processing the input audio signal and an output unit for providing an audible signal to the user in dependence on the processed audio signal.

The input unit may include multiple input microphones, e.g. for providing direction-dependent audio signal processing. Such directional microphone system is adapted to enhance a target acoustic source among a multitude of acoustic sources in the user's environment. In one aspect, the directional system is adapted to detect (such as adaptively detect) from which direction a particular part of the microphone signal originates. This may be achieved by using conventionally known methods. The signal processing unit may include amplifier that is adapted to apply a frequency dependent gain to the input audio signal. The signal processing unit may further be adapted to provide other relevant functionality such as compression, noise reduction, etc. The output unit may include an output transducer such as a loudspeaker/ receiver for providing an air-borne acoustic signal transcutaneously or percutaneously to the skull bone or a vibrator for providing a structure-borne or liquid-borne acoustic signal. In some hearing devices, the output unit may include one or more output electrodes for providing the electric signals such as in a Cochlear Implant.

A Cochlear Implant typically includes i) an external part for picking up and processing sound from the environment, and for determining sequences of pulses for stimulation of the electrodes in dependence on the current input sound, ii) a (typically wireless, e.g. inductive) communication link for simultaneously transmitting information about the stimulation sequences and for transferring energy to iii) an implanted part allowing the stimulation to be generated and applied to a number of electrodes, which are implantable in different locations of the cochlea allowing a stimulation of different frequencies of the audible range. Such systems are e.g. described in US 4,207,441 and in US 4,532,930.

In an aspect, the hearing device comprises multi-electrode array e.g. in the form of a carrier comprising a multitude of electrodes adapted for being located in the cochlea in proximity of an auditory nerve of the user. The carrier is preferably made of a flexible material to allow proper positioning of the electrodes in the cochlea such that the electrodes may be inserted in cochlea of a recipient. Preferably, the individual electrodes are spatially distributed along the length of the carrier to provide a corresponding spatial distribution along the cochlear nerve in cochlea when the carrier is inserted in cochlea.

Referring now to Figure 2A and 2B, schematic views of a hearing aid 200 according to an embodiment is illustrated. The implementation of the hearing aid 200 may be similar to the implementation of the hearing aid described in connection with Fig. 1. Various electrical components 110 are arranged in two cavities 242, 252. A first part 240 of a one-piece PCB (e.g. with additional electrical components at the front and/or bottom side) forms a cavity enclosure component 270 of a cavity enclosure structure of a rear cavity 242 and a second part 250 of the one-piece PCB (e.g. with additional electrical components at the front and/or bottom side) forms a cavity enclosure component of a cavity enclosure structure of a front cavity 252. The one-piece PCB is illustrated in an exploded view (e.g. substrate is lifted from the cavity). A cavity enclosure component 270 of the cavity enclosure structure enclosing the rear cavity 242 comprises a rear injection hole 244 and at least two inspection openings 246, 248 (e.g. inspection hole or ventilation gap). Further, the cavity enclosure component 270 also forms part of a cavity enclosure structure enclosing the front cavity 252 and comprises a front injection hole (not shown) and a front inspection opening (e.g. an inspection hole or ventilation gap, not shown). The cavity enclosure component 270 is arranged in a shell component 260 of the hearing aid 200. The implementation of the injection and inspection holes can enable a reliable injection of filling material into the cavities to fill at least the space between one side (e.g. front side or back side) of the PCBs and the cavity enclosure structure or substantially the whole space not occupied by components of the hearing aid within the front and rear cavity.

Fig. 2A and 2B show an example with two injection holes (one for each of the two cavities) and five inspection holes. In this example, only the lower side of the PCB is coated by using the disclosed cavity conformal coating technique. The coating may be forced into the cavity by pressure from a e.g. an injection needle, while at the same time, it may be sucked into the cavity by capillary forces (e.g. like water sucking up in the small cavities in a piece of kitchen towel). This may make sure that all cavities are filled with the filling material, e.g. to provide a protective coating of the electrical components in the cavity.

Large air traps in the cavity may be avoided by avoiding having dead ends from where the air cannot be pushed out of any ventilation holes or be pushed in front of the coat's float front and out of the inspection holes. The ventilation holes may be made by leaving a minor gap between the PCB and the Chassis. This gap may be small enough to prevent the coat from flowing out since the coat will be held back by the capillary forces in the narrowest section of the small gaps so the coat does not tend to continue its flow through the gap, for example.

In an example embodiment, the cavities may be provided inside a part of the housing structure, e.g. a chassis enclosed by a shell structure, where a system of grooves and holes may lead the coating from the injection hole to the inspection holes provided in the chassis (e.g. Fig. 2A). However, at least the first opening, i.e. the inspection hole may also be provided in the shell structure and/or in the substrate.

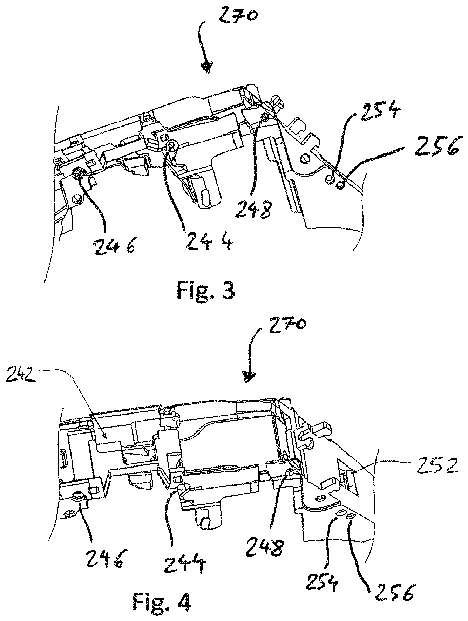

Fig. 3 and 4 show schematic views of the cavity enclosure component 270 of the hearing aid 200 with the rear injection hole 244 and the two inspection openings 246, 248 as well as the front injection hole 254 and the front inspection opening 256.

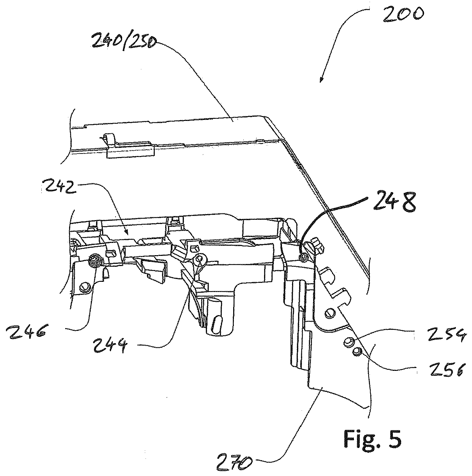

Figure 5 illustrates a schematic view of the substrate (e.g. one-piece PCB comprising first part 240 and second part 250) and the cavity enclosure component 270 (e.g. part of the housing structure or chassis) of the hearing device 200.

More details and aspects are mentioned in connection with the embodiments described above or below. The embodiment shown in Figs. 2A to 5 may comprise one or more optional additional features corresponding to one or more aspects mentioned in connection with the proposed concept or one or more embodiments described above (e.g. Figs. 1) or below.

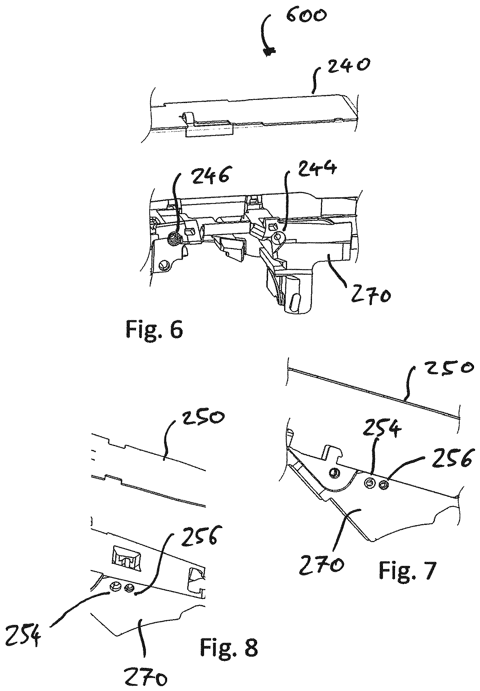

Figure 6 illustrates a schematic view of a substrate 240 (e.g. first PCB) and a cavity enclosure component 270 of a hearing aid 600 according to an embodiment. The implementation of the hearing aid 600 may be similar to the implementation of the hearing aid described in connection with Fig. 2A-5. However, the one-piece PCB partly enclosing the first cavity and the second cavity of the hearing aid described in connection with Fig. 2A-5 is implemented by two separate PCBs 240, 250.

Figure 7 and 8 illustrate other schematic views of the cavity enclosure component 270 of the hearing aid 600 together with the second PCB 250.

More details and aspects are mentioned in connection with the embodiments described above or below. The embodiment shown in Figs. 6-8 may comprise one or more optional additional features corresponding to one or more aspects mentioned in connection with the proposed concept or one or more embodiments described above (e.g. Figs. 2A-5) or below.

Fig. 9 illustrates a schematic cross section of a cavity of a hearing aid 900. The implementation of the hearing aid 900 may be similar to the implementation of the hearing aid described in connection with Fig. 1. A plurality of electrical components 110 are arranged on a PCB 206 forming a first part of the cavity enclosure structure. A second part of the cavity enclosure structure is formed by a housing component 208. The PCB fits into a recess of the housing component 208 with a low tolerance C. This tolerance C is merely provided for allowing minor changes in the substrate structure (i.e. the PCB 206) to be connected to the cavity. The injection hole 122 comprises a conical geometry at an end located at the outside surface to enable an easy insertion of the injection needle 209 to the injection hole 122 and/or a capillary force attracting the filling material dispensed by the injection needle 209. The conical shaping of the injection hole also provides a good sealing between the injection needle and the injection hole such that the filling material is not allowed to flow out of the injection hole during the injection of the filling material.

The inspection holes 124 comprise different diameters. The inspection hole 124 located closer to the injection hole 122 comprises a diameter E smaller than a diameter A of the inspection hole 124 located farther away from the injection hole 122. In this way, a leaking of filling material out of the closer inspection hole may be slowed in comparison to the other inspection hole so that the filling material leaks out of both inspection holes and can be detected approximately at the same time. Accordingly, the design of the openings 124 may be such that the filling material reach into the opening (i.e. the inspection holes 124) at the same time, since the cappilary forces of the openings differs along with the different designs. The inspection holes 124 comprise a step-shaped increase of the diameter of the opening 124 at the outside surface to stop the capillary effect, for example. That is, the inspection holes 124 has a transition from a narrow path (defined by A) to a wider path located closer to the outside of the hearing aid shell, chassis or housing structure. This widening provides the possibility of collecting any overflow of filling material which is forced out the inspection opening. Furthermore, the transition comprises a sharp edge, which has the function of a capillary trap that forces the filling material to stop it flow out of the inspection hole 124.

A part of the cavity located close to the injection hole 122 comprises a first height D (or diameter) and a part of the cavity located close to the farther away opening 124 comprises a second height B (or diameter). The second height B is smaller than the first height D and the cavity narrows along the path from the first height D to the second height B, which forces the filling material in the direction towards the smaller second height B, due to capillary forces acting on the filling material.

To help the filling material flow all the way into the cavity, the diameter of a inscribed circle at the very end of the cavity (distance B) is smaller than the diameter of a inscribed circle at the very beginning of the cavity (distance D). As long as this principal may be followed as a guide line, the filling flows as good as practically possible. This may be to utilize the capillary action to help the filling material flow.

For example, the surfaces of the components and housing etc. in contact with the filling material may be "filling material phallic" at the moment of the filling process. Meaning that the contact angle of a droplet of the filling material placed on a flat surface of e.g. the housing is smaller than 90 degrees. A contact angle above 90 degrees would prevent capillary forces to interact with the filling material. The external opening of the injection hole 122 can have a conic shape allowing a nozzle 209 (e.g. an injection needle) in a plurality of sizes to seal sufficiently to the same opening. The sealing points/ring may vary in position depending on the size of the nozzle.

The conic shape 201 may further also help the operator/automated dispensing mechanism to hit the injection hole quick and correctly, since the conic sides will guide the nozzle (e.g. the injection needle).

With the cavity being created with a slight slope from the injection hole in a first end to a second end of the cavity, the filling material will have a tendency to "crawl" down the slope and reach the entire cavity, for example.

Figure 10 illustrates a schematic cross section of an inspection hole of a hearing aid 1000. The implementation of the hearing aid 1000 may be similar to the implementation of the hearing aid described in connection with Fig. 1 or 9. The cavity may comprise a substantially constant height B (or varying height as in Fig. 9). The inspection hole 124 comprises an inner sharp edge 205 towards the cavity 201. The inspection hole 124 can have a first sharp edge 202 and an overflow cavity 203 followed by a second sharp edge 204 to stop the flow of filling material by the mean of capillary forces as soon filling material reaches the first sharp edge 202. In case of too much filling material is injected the overflow cavity will be utilized. Overfilling the overflow cavity is partially prevented by the second sharp edge, due to the sharp edge creating a capillary trap, in the form a capillary stop, which prohibits the filling material to flow any further.

To make sure that filling material gets visual in the inspection hole, the diameter of the inscribed circle of the inspection hole size A may be smaller than the inscribed circle B of the cavity 201. This may by the means of capillary forces suck the filling material into the inspection opening. The inner edge of the inspection hole 205 may be rounded.

To prevent filling material to flow onto the PCB 206, the inscribed circle of the opening C may be significantly smaller than both A and B. For example, C is as small as possible. This is to trap the filling material in the opening by the means of capillary forces. C may also work as a ventilation opening and clearance tolerance variation in assemblying of the PCB and housing structure, for example.

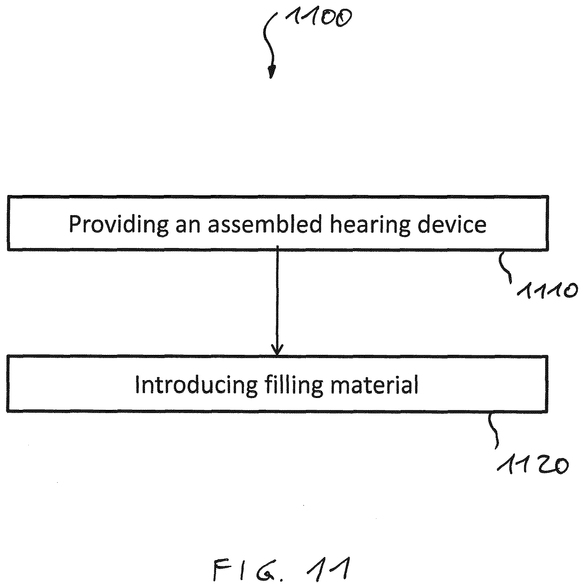

Fig. 11 shows a flow chart of a method for producing a hearing aid according to an embodiment. The method 1100 comprises providing 1110 an at least partly assembled hearing aid comprising an electrical component arranged in a cavity and a cavity enclosure structure enclosing the cavity. The cavity enclosure structure comprises an injection hole and an opening. Further, the injection hole and the opening are formed between an outside surface of the cavity enclosure structure and the cavity. Further, the method 1100 comprises introducing 1120 filling material through the injection hole into the cavity at least until the filling material is detectable through the opening.

The method 1100 may further comprise assembling the hearing aid before injecting 1120 the filling material.

Additionally, the method 1100 may comprise detecting the filling material (e.g. by an optical sensor) through or at the opening and stopping introducing (e.g. injecting) filling material into the injection hole when the filling material is detected.

More details and aspects are mentioned in connection with the embodiments described above or below. The embodiment shown in Fig. 11 may comprise one or more optional additional features corresponding to one or more aspects mentioned in connection with the proposed concept or one or more embodiments described above (e.g. Figs. 1-10) or below.

Embodiments of the disclosure relate to injection holes and one or more openings for conformal coating of hearing instruments. A conformal coating of hearing aids may enable a protection of the electronic components from corrosive liquids, to protect the electronics from electro static discharge, to seal some connections, making them air or sound tight and/or to fix some of the components to each other making the instrument more robust to impact.

In contrast to a coating by having a locally applied coating just covering the individual components, and dispensed with a syringe, the filling of cavities may be improved by the proposed concept. For example, it may be avoided that the coating of other concepts is time consuming to apply, since the needle of the syringe needs to be moved around to many locations on the electronics (e.g. which may be no matter if it is done as an automatic or a manual process), is difficult to control where it moves after it is applied till it has hardened and/or it requires a lot of training to be able to get a consistent applied coating from a manual process. Some operators may apply more and in large areas, other operators may apply less in only very small areas.

According to an embodiment, some of the ventilation gaps may be made smaller since coat may be running out through some of them (otherwise).

According to an embodiment, a reservoir (e.g. recess in the outside surface) around the injection holes (and some of the inspection holes) may be implemented to contain small droplet sitting in the end of the coating needle before coating starts and to contain coat that exceeds the inspection holes, for example.

According to another embodiment, a balance between inspection holes may be improved to make the coat be visible through all the holes in the chassis at some what the same time. For example, by balance may be meant that the different inspection holes may be designed in different sizes, with regards to diameter across a cross-section of the opening, such that the filling material having a tendency to flow into all inspection holes at the same time. That is the filling material will be more prone to flow through a large cross-sectional area than to a narrow inspection hole having a small cross-section area. Therefore, if providing e.g. two inspection holes whit different sized cross-sections, the filling material may have a tendency to reach a second inspection hole with a larger cross-section than a first inspection hole, prior to reaching the outside surface of the first inspection opening.

An aspect of the proposed concept may be to arrange the PCB in a housing structure of the hearing aid (e.g. in between two Chassis parts or other housing parts, such as a shell structure) and apply the coating through one hole that seals to the needle of the syringe. When the coating is visible through an inspection hole, implemented as at least one first opening in the housing structure, the substrate of the e.g. the chassis, the coating process may be finished. There may be one or more inspection holes for the coating. The cavity may even be made as a completely closed cavity around the full PCB or it may be created by holding one side of PCB tight against a cavity in e.g. the chassis part (e.g. making the PCB function as the lid for the cavity) and apply coating into the cavity and thereby letting it cover only the lower side of the PCB.

In an embodiment, the process of injecting the filling material into the cavities of the hearing device, such as a hearing aid, may be automated. That is, in an embodiment a set of instruction functions may be stored on or encoded as one or more instructions on a computer connected to a manufacturing device, which comprises an injection tool. The instruction functions may be configured to run a computer program which controls the injection process, by instructing the amount of filling material, controls the injection tool and/or controls the process of filling the cavity. For example, the injection of the filling material may be controlled by a software running on a processor of an injection system.

It is intended that the structural features of the devices described above, either in the detailed description and/or in the claims, may be combined with steps of the method, when appropriately substituted by a corresponding process.

As used, the singular forms "a," "an," and "the" are intended to include the plural forms as well (i.e. to have the meaning "at least one"), unless expressly stated otherwise. It will be further understood that the terms "includes," "comprises," "including," and/or "comprising," when used in this specification, specify the presence of stated features, integers, steps, operations, elements, and/or components, but do not preclude the presence or addition of one or more other features, integers, steps, operations, elements, components, and/or groups thereof. It will also be understood that when an element is referred to as being "connected" or "coupled" to another element, it can be directly connected or coupled to the other element but an intervening elements may also be present, unless expressly stated otherwise. Furthermore, "connected" or "coupled" as used herein may include wirelessly connected or coupled. As used herein, the term "and/or" includes any and all combinations of one or more of the associated listed items. The steps of any disclosed method is not limited to the exact order stated herein, unless expressly stated otherwise. Furthermore, the wording "filled" could mean" filled" or "at least partly filled" or "possibly filled" or "occasionally filled".

It should be appreciated that reference throughout this specification to "one embodiment" or "an embodiment" or "an aspect" or features included as "may" means that a particular feature, structure or characteristic described in connection with the embodiment is included in at least one embodiment of the disclosure. Furthermore, the particular features, structures or characteristics may be combined as suitable in one or more embodiments of the disclosure. The previous description is provided to enable any person skilled in the art to practice the various aspects described herein. Various modifications to these aspects will be readily apparent to those skilled in the art, and the generic principles defined herein may be applied to other aspects.

The claims are not intended to be limited to the aspects shown herein, but is to be accorded the full scope consistent with the language of the claims, wherein reference to an element in the singular is not intended to mean "one and only one" unless specifically so stated, but rather "one or more." Unless specifically stated otherwise, the term "some" refers to one or more. Accordingly, the scope should be judged in terms of the claims that follow.

## Claims

1. A hearing device comprising:
at least one electrical component arranged in a cavity, wherein the cavity is filled at least partly with a filling material; and
a cavity enclosure structure enclosing the cavity, wherein the cavity enclosure structure comprises an injection hole at least partly filled with the filling material and an opening, wherein the injection hole and the opening are formed between an outside surface of the cavity enclosure structure and the cavity, wherein the filling material extends at least from the injection hole towards the opening through the cavity so that the filling material is detectable through the opening.

2. Hearing device according to claim 1, wherein the filling material fills more than 70% of a space of the cavity unoccupied by components of the hearing device.

3. Hearing device according to one of the previous claims, wherein a path extends in the interior of the cavity between the injection hole and the opening, wherein the path at a part of the cavity located close to the injection hole comprises a first height and the path at a part of the cavity located close to the opening comprises a second height, wherein the second height is smaller than the first height, wherein the cavity narrows along the path from the first height to the second height.

4. Hearing device according to one of the previous claims, wherein the opening is one inspection opening of a plurality of the inspection openings of the cavity enclosure structure, wherein the filling material is detectable through each inspection opening of the plurality of the inspection openings.

5. Hearing device according to one of the previous claims, wherein the injection hole is formed with a substantial conical geometry, such that the largest diameter of the injection hole is formed at the outer surface of the cavity enclosure structure and the injection hole narrows in diameter towards the interior of the cavity.

6. Hearing device according to one of the previous claims, wherein at least one of the injection hole and the opening is formed as a recess at the outside surface of the cavity enclosure structure.

7. Hearing device according to one of the previous claims, wherein the opening is configured with a path extending from the interior of the cavity to an outside of the cavity enclosure structure, wherein the path narrows in cross-section area from the interior of the cavity to an end facing a transition to a last part of the opening facing the outside of the cavity enclosure structure, wherein the transition is formed as a widening of the cross-section of the path.

8. Hearing device according to one of the previous claims, wherein the at least one electrical component is arranged on a substrate, wherein the substrate is a second cavity enclosure component of the cavity enclosure structure connected to a first cavity enclosure component of the cavity enclosure structure, wherein the cavity enclosure components of the cavity enclosure structure together enclose the cavity.

9. Hearing device according to one of the previous claims, wherein the at least one electrical component is carried by a substrate configured as a printed circuit board.

10. Hearing device according to one of the previous claims, wherein the cavity enclosure structure comprises a plurality of cavity enclosure components assembled to form the cavity enclosure structure, wherein the opening is provided as a gap between two cavity enclosure components of the cavity enclosure structure.

11. Hearing device according to one of the claims 1 to 9, wherein the injection hole and the opening are formed in the same cavity enclosure component of the cavity enclosure structure.

12. Hearing device according to one of the previous claims, wherein the injection hole comprises a cross section with a maximal extension of less than 2mm.

13. Hearing device according to one of the previous claims, wherein a further electrical component is arranged in a further cavity filled at least partly with further filling material, wherein a further cavity enclosure structure encloses the further cavity, wherein the cavity enclosure structure comprises a further injection hole at least partly filled with the filling material and a further opening, wherein the filling material extends at least from the further injection hole towards the further opening through the further cavity so that the further filling material is detectable through the further opening.

14. Hearing device according to one of the previous claims, wherein the electrical component is at least one of a signal processor circuit, a filter circuit, an analog-to-digital converter circuit, a memory circuit, a sensor circuit, a digital-to-analog converter circuit, a receiver circuit, a transmitter circuit, a transceiver circuit, a capacitor and an inductor.

15. A method for producing a hearing device, the method comprising:
providing an at least partly assembled hearing device comprising an electrical component arranged in a cavity and a cavity enclosure structure enclosing the cavity, wherein the cavity enclosure structure comprises an injection hole and an opening, wherein the injection hole and the opening are formed between an outside surface of the cavity enclosure structure and the cavity; and
introducing filling material through the injection hole into the cavity at least until the filling material is detectable through the opening.
